# EUROPEAN PATENT APPLICATION

(11) **EP 1 306 672 A2**
(43) Date of publication of application: **02.05.2003**
(21) Application number: 02090361.3
(22) Date of filing: 23.10.2002
(51) Int. Cl.: G01N 33/48

(54) **Method of screening drug for hepatitis C**

(30) Priority: 26.10.2001 JP 2001329728
(71) Applicant: BML, Inc., Tokyo 151-0051 (JP); MAX-DELBRÜCK-CENTRUM FÜR MOLEKULARE MEDIZIN, 13125 Berlin (DE)
(72) Inventor: Fukushi, Shuetsu, Kawagoe-shi, Saitama 350-1101 (JP); Kageyama, Tsutomu, Kawagoe-shi, Saitama 350-1101 (JP); Hoshino, Fuminori, Kawagoe-shi, Saitama 350-1101 (JP); Katayama, Kazuhiko, c/o National Institute of, Musashimurayama-shi, Tokyo 208-0011 (JP); Stahl, Joachim, 14195 Berlin (DE); Okada, Masato, c/o Research Institute for, Suita-shi, Osaka 565-0871 (JP)
(74) Representative: Ziebig, Marlene, Dr. Dipl.-Chem.

(57) **Abstract**

Hepatitis C virus-biding protein has been found to be a 40S ribosomal protein S5 composing a 40S ribosomal subunit of an eukaryote. On the basis of this finding, there is provided a method for screening test substances for a substance exhibiting anti-hepatitis-C-virus activity, through detection of inhibitory activity of the substance against binding between an IRES of a hepatitis C virus gene and a 40S ribosomal protein S5.

## Description

### Background of the Invention

### Field of the Invention

The present invention relates to means for screening substances, and more particularly, to means for screening substances for potential therapeutic drugs for hepatitis C.

### Description of the Related Art

In Japan, cases of hepatitis C account for more than 90% of cases of post-transfusion non-A non-B hepatitis. Because of delays in establishing therapies therefor, hepatitis C, a type of non-A non-B hepatitis, is now responsible for 70 to 80% of the causes of onset of liver cancer.

Presently, an interferon therapy is accepted as a mainstream approach for the treatment of hepatitis C.

According to the interferon therapy, interferon products which have been approved as drugs for the treatment of chronic hepatitis C are administered to patients over six months.

However, interferon is known to be accompanied by a variety of side effects, including fever, systemic malaise, headache, arthralgia, inappetence, palpitation, numbness of the extremities, dizziness, falling of hair, and depression. Moreover, interferon does not exhibit any appreciable effect when administered to patients suffering hepatitis C type 1b, which account for two out of every three Japanese hepatitis C patients. In addition, interferon fails to exhibit satisfactory effect on advanced hepatitis C patients.

Thus, therapeutic means for hepatitis C has not yet been established to a satisfactory level, and epoch-making therapeutic means is awaited.

If a substance capable of preventing multiplication of a hepatitis C virus by blocking the multiplication mechanism of the virus were discovered, such a substance would prove to be a promising therapeutic means for hepatitis C. However, at present, the multiplication mechanism of the hepatitis C virus has not yet been elucidated completely, and thus no effective method for screening potential anti-virus agents on the basis of such a mechanism has been obtained.

### Summary of the Invention

An object of the present invention is to provide, through elucidation of the multiplication mechanism of the hepatitis C virus, means for screening substances; specifically, means capable of screening substances for a substance having anti-hepatitis C virus activity, on the basis of the multiplication mechanism.

The present inventors had previously studied hepatitis C virus binding protein, and have filed a patent application related thereto (Japanese Patent Application Laid-Open (*kokai*) No. 10-204100). The inventors have extended their study regarding the hepatitis C virus binding protein, and quite surprisingly, have found that this virus binding protein is in fact a 40S ribosomal protein S5, which is a constituent of a 40S ribosomal subunit of an eukaryote (as will be described hereinafter in the "Examples" section).

Since the step of binding the 40S ribosomal protein S5―which functions as a hepatitis C virus binding protein―to an IRES of hepatitis C virus is indispensable for multiplication of the hepatitis C virus in a host, the present inventors conceived that the mentioned binding may be used as an index in the screening of substances for anti-hepatitis-C-virus activity. (Note that IRES is an abbreviation of "internal ribosome entry site." An IRES is present in the 5' non-translation region of a gene of an RNA virus, such as hepatitis C virus. Upon expression of the gene, ribosome is first bound to this site. See Tsukiyama-Kohara *et al*., J. Virol., 66, 1476-1483 (1992); and Wang *et al*., J. Virol., 67, 3338-3344 (1993).)

Accordingly, the present invention provides a method for screening test substances for a substance exhibiting anti-hepatitis-C-virus activity, through detection of inhibitory activity of the substance against binding between an IRES of a hepatitis C virus gene (unless otherwise specified, hereinafter "IRES" is used to refer to an IRES of a hepatitis C virus gene) and a 40S ribosomal protein S5 (hereinafter the method may be called "the present screening method").

According to the method of the present invention, when any inhibitory effect that prevents binding of IRES to 40S ribosomal protein S5 is detected in a test substance, the test substance is predicted to exhibit an effect of preventing multiplication of hepatitis C virus through inhibitory action exerted on the binding, and thus the test substance is considered a candidate for an active ingredient to be contained in a therapeutic drug for the treatment of hepatitis C.

In the present screening method, no particular limitation is imposed on the test substances to be screened; any types of substance, such as organic compounds, inorganic compounds, substances of natural origin, or nucleic acid, may be employed, so long as they may be used in studies for anti-hepatitis C virus activity.

As used herein, "kDa (kilo-dalton)" refers to a unit of weight used to express molecular weight, wherein 1 kDa corresponds to a 1,000-fold value of the mass of one hydrogen atom (1.67 × 10⁻²⁴ g).

Hereinafter, hepatitis C virus may be referred to as HCV, and 40S ribosomal protein S5 may be referred to as rpS5.

### Brief Description of the Drawings

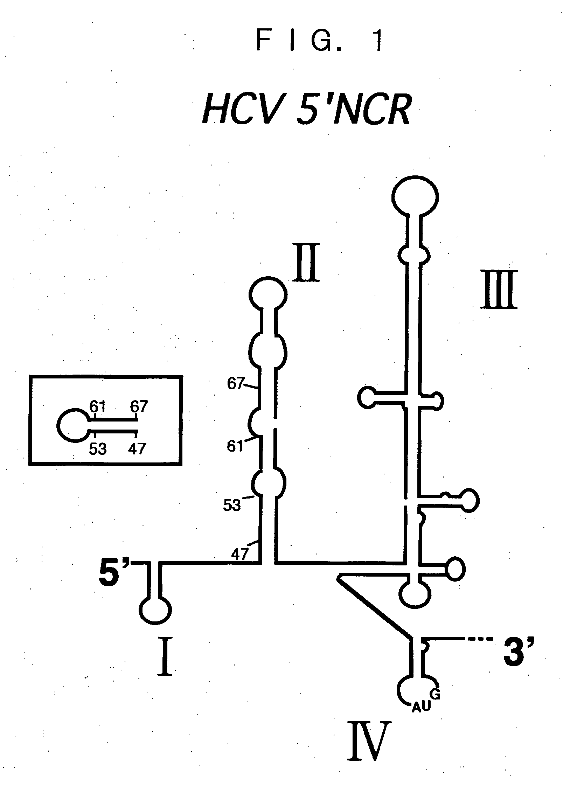

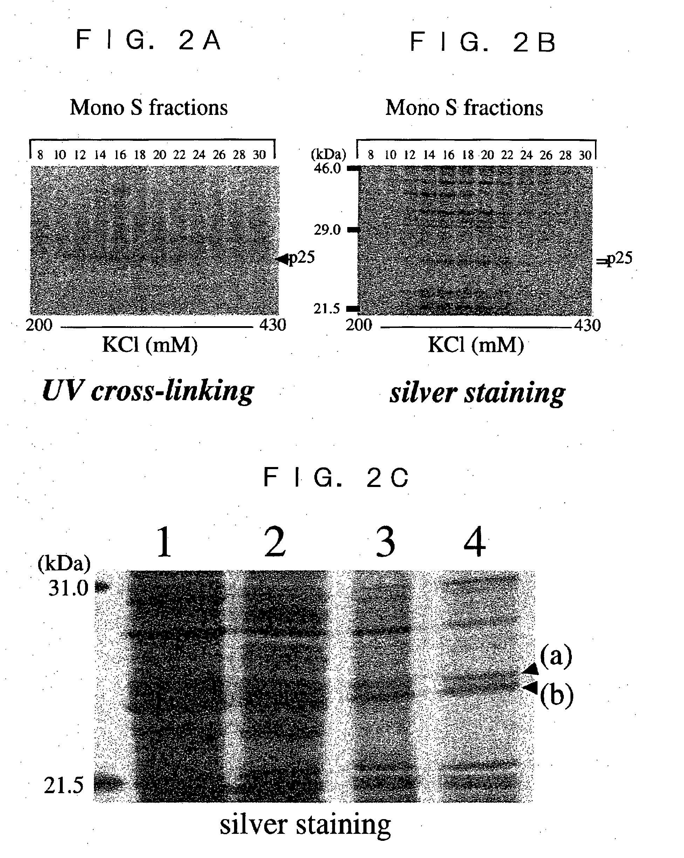

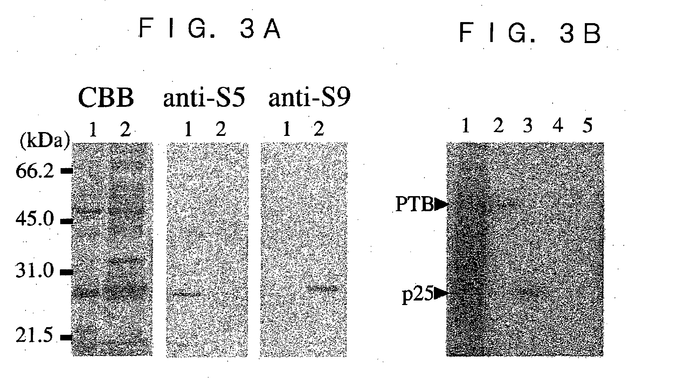

Fig. 1 shows the secondary structure of the 5' non-translation region of HCV gene; Figs. 2A to 2C show the results obtained from isolation of p25 that has been cross-linked with HCV IRES; and Figs. 3A and 3B show the results of identification test of p25.

### Detailed Description of the Preferred Embodiment

Modes for carrying out the present invention will next be described.

### IRES

As described above, an IRES is essentially a structure found in the 5' non-translation region of a HCV gene and is formed of a polynucleotide that is indispensable for initiation of translation of the virus gene. The nucleotide sequence of the 5' non-translation region of a wild-type HCV gene has already been reported (Inchauspe *et al*., Proc. Natl. Acad. Sci. USA., 88, 10292-10296 (1991)), and is specifically represented by the nucleotide sequence (cDNA) of SEQ ID NO: 1.

Needless to say, the IRES which may be employed in the present invention may be the entirety of the polynucleotide constituting the nucleotide sequence represented by SEQ ID NO: 1. However, the IRES may be a portion of the polynucleotide represented by SEQ ID NO: 1 or may be a modified sequence obtained through addition or alteration, so long as such IRES has a function of initiating translation of the HCV gene.

The 5' non-translation region of a HCV gene has a secondary structure as shown in Fig. 1 (Honda *et al*., J. Virol., 73, 1165-1174 (1999)). In relation to Fig. 1, the portion corresponding to the 47th to 67th bases may form a stem-loop structure as shown in the square window (Brown *et al*., Nucleic Acids Res., 20, 5041-5045 (1992)). In Fig. 1, the stem-loop structure in region II is primarily responsible for initiation of translation for an HCV gene (in the case of wild-type HCV, the stem-loop structure corresponds to the 47th to 67th bases in the nucleotide sequence of SEQ ID NO: 1), and in particular, the stem portions that neighbor the loop portion play the most important role. Therefore, preferably, the IRES employed in the present invention has a stem-loop structure which corresponds to the 47th to 67th bases in the nucleotide sequence represented by SEQ ID NO: 1 of wild-type HCV. More preferably, the stem portions corresponding to the 47th to 53rd bases and the 61st to 67th bases in the nucleotide sequence represented by SEQ ID NO: 1 are identical with the corresponding sequences in SEQ ID NO: 1. The portion corresponding to the loop portion; i.e., the 54th to 60th bases in SEQ ID NO: 1, may undergo any nonessential alteration (to an extent such that the loop structure is maintained), since such alteration provides no substantial effect on the translation initiation function on HCV. Therefore, such nonessential alteration in nucleotide sequence is permissible for the IRES employed in the present invention.

So long as the stem-loop structure, particularly the stem portions of the structure, is conserved, other portions (specifically, in the 1st to 342nd base sequence of the polynucleotide that constitutes the 5' non-translation region of wild-type HCV, portions other than the sequence corresponding to the 47th to 67th bases) may undergo any arbitrary change in base (the change including addition and/or deletion of bases) to an extent such that no substantial adverse effect is exerted on physical/chemical properties of the stem-loop structure, particularly the stem portions of the structure, and such modified structure may be employed as the IRES as defined by the present invention.

The IRES may be prepared as follows. Briefly, by employment of HCV cDNA as a template, a gene amplification technique, such as PCR, is carried out so that the promotor sequence of RNA polymerase is added to the 5' terminal of the IRES sequence, to thereby yield a gene amplification product. The product is purified, and through use of the thus-purified product as a template, together with RNA polymerase, an RNA synthesis reaction is carried out. The resultant RNA is purified through a phenol extraction or a column procedure such as gel filtration. The thus-purified RNA may be used as the IRES. Alternatively, RNA having a nucleotide sequence that constitutes the IRES of interest may be chemically synthesized and employed.

If necessary, the thus-prepared IRES may be labeled, and the labeled IRES employed as the IRES of the present invention. The labeling substance may be any of those employed for labeling nucleic acid and in particular RNA. Specifically, radioisotope labels such as ³²P, ³³P, ³⁵S, ¹²⁵I, and ¹³¹I may be used in accordance with a routine method.

### rpS5

The rpS5 which may be employed in the present invention can be prepared according to a conventional method.

Specifically, rpS5 of interest can be prepared through either of the following processes (i) and (ii). In process (i), a ribosome fraction is isolated from an eukaryote cell culture or liver tissue, or other tissue of a mammal; the 40S subunit and the 60S subunit are removed from the ribosome fraction, to thereby select solely the 40S subunit; and from this 40S subunit, rpS5 is isolated (this process is called the isolation method). In process (ii), a gene coding for rpS5 is isolated, and the gene is caused to be expressed, whereby a recombinant rpS5 is produced (this process is called the recombinant method).

### (i) Isolation method

No particular limitation is imposed on eukaryote cells which may be used in the aforementioned isolation method, and there may be employed yeast, cells derived from a mammal, and insect cells. In view that the substances to be screened according to the present screening method are pharmaceutically active substances for humans, generally, use of mammal cells, in particular cultured cells derived from a human, is preferred. Specific examples include HeLa cells, HepG2 cells, and MT-2 cells. From such eukaryote cells, a cell extract is prepared by a routine method, and the cell extract is subjected to ultra-centrifugation (27,000 ×g or thereabouts), whereby a ribosome fraction is obtained. The ribosome fraction is subjected to treatment with puromycin and potassium chloride to thereby separate 40S subunit and 60S subunit, for collection of solely 40S subunit. The isolated 40S subunit is subjected to treatment with potassium chloride, ion exchange chromatography, or a similar treatment, whereby rpS5 is obtained for use in the present invention.

In the screening method of the present invention, rpS5 as contained in the 40S ribosomal subunit (i.e., rpS5 in a state in which it composes a portion of the 40S ribosomal subunit) may be used. Thus, the isolation method is suitable for preparing the rpS5 to be used in such a mode of the present screening method.

### (ii) Recombinant method

A gene coding for rpS5 is disclosed in, for example, J. Biol. Chem., 267, 25304-25308 (1992) (Kuwano *et al*.). Therefore, with reference to teachings of such a reference, a gene coding for rpS5 can be effectively prepared, and through expression of the gene, rpS5 can be prepared.

Specifically, a gene amplification method such as PCR is performed using , as amplification primers, of nucleotide sequences which are complimentary to the sequences of the both terminals of the gene coding for rpS5, to thereby prepare a gene amplification product of the gene coding for rpS5. The thus-prepared gene amplification product is inserted into an appropriate vector. A host carrying the vector is used as a transformant, in which the gene coding for rpS5 is expressed, whereby a recombinant rpS5 of interest can be prepared.

The recombinant method is suitable in another mode of the present screening method, where rpS5 *per se* is used; i.e., where rpS5 is not in "as-contained" form in the 40S ribosomal subunit.

The rpS5 obtained through either one of the above methods may be isolated and purified through use of conventionally known isolation/purification means, such as isoelectric point electrophoresis or gel filtration, before use in the present screening method.

If necessary, the thus-obtained rpS5 may be labeled, and the labeled rpS5 may be used as the rpS5 of the present invention. The labeling substance may be any of those employed for labeling nucleic acid and in particular protein. Specifically, radioisotope labels such as ³²P, ³³P, ³⁵S, ¹²⁵I, and ¹³¹I may be used in accordance with a routine method.

### Modes of the present screening method

As described above, in the present screening method, detection of inhibitory effect of a test substance on binding of IRES to rpS5 is essential.

The IRES and rpS5 employed in the detection system of the present invention may be prepared as described hereinabove.

According to the present screening method, the inhibitory effect that prevents binding of IRES to rpS5 can be detected through, for example, the UV cross-linking method, gel mobility shift assay, or the surface plasmon resonance method.

The UV cross-linking method is a typical method for detecting a nucleic acid binding protein.

Briefly, when a protein - RNA complex is irradiated with UV light, covalent bonding occurs between the protein and RNA. In the case where binding between protein and RNA is such that the protein recognizes a certain specific site of labeled RNA, selective labeling of RNA binding protein can be obtained. According to the UV cross-linking method, the molecular weight of the labeled RNA binding protein is checked through electrophoresis, to thereby detect the RNA binding protein.

When the UV cross-linking method is employed as detection means, it is possible to determine whether or not rpS5 is bound to IRES, for example, as follows. A test substance, a labeled IRES, and rpS5―which is an RNA-binding protein―are subjected to incubation under conditions that would naturally induce binding between IRES and rpS5, followed by irradiation with UV light for cross-linking. The resultant cross-linked substance is electrophoresed, and a check is made as to whether a protein-attributed band is observed at a position corresponding to the molecular weight of rpS5. When binding between IRES and rpS5 is detected, it follows that the test substance does not have the effect of preventing binding between IRES and rpS5, whereas when binding between IRES and rpS5 is not detected, it follows that the test substance prevents binding between IRES and rpS5.

Binding of IRES to rpS5 is an indispensable step for initiation of translation of a HCV gene. Therefore, if a test substance exhibits an effect of preventing binding between IRES and rpS5, observation of such an effect is indicative that the test substance has an effect of preventing multiplication of HCV in the host through prevention of translation of the gene in the virus. Thus, the test substance becomes to be a candidate substance to be selected in the screening.

According to the gel shift assay, protein and labeled RNA are incubated, and the incubation mixture is directly applied onto a gel for polyacrylamide gel electrophoresis (PAGE) under non-denaturing conditions, whereby whether or not RNA binds to protein is detected on the basis of any difference in electrophoresis migration distance indicated by labeled RNA. When the gel shift assay is employed as detection means, whether rpS5 is bound to IRES can be determined, for example, as follows. A test substance, a labeled IRES, and rpS5 which is an RNA-binding protein, are subjected to incubation under conditions that would naturally induce binding between IRES and rpS5, followed by PAGE under non-denaturing conditions. On the basis of checking as to whether or not electrophoresis migration distance varies between the incubation mixture and RNA that has not been bound to protein, presence or absence of binding between rpS5 and IRES can be determined. That is, when any change in migration distance is detected, it follows that formation of a complex formed by binding of rpS5 to IRES is confirmed, whereas when no such change is detected, it follows that binding of rpS5 to IRES has been prevented by the test substance.

According to the surface plasmon resonance method, RNA (or a protein) is applied at a certain flow rate onto a sensor chip to which gold has been deposited to form thin film. Any change in mass that would occur on the surface of the sensor chip is transformed to optical signals of surface plasmon resonance (SPR signals), and the signals are detected. Thus, when the surface plasmon resonance method is employed, for example, IRES is immobilized onto a sensor chip, and when a test substance and rpS5 are added thereto, any change in SPR signal is detected, whereby presence or absence of binding between rpS5 and IRES is determined.

Alternatively, when inhibitory effect on HCV translation activity, which is caused by inhibited binding of rpS5 to IRES, is employed as an index, a determination can be made as to whether the mentioned inhibited binding of rpS5 to IRES is present or absent in the test substance. For example, when a test substance is added to an in vitro HCV translation reaction mixture and the translation reaction activity level drops, the test substance is found to have an activity of inhibiting binding between rpS5 and IRES, whereas when translation reaction activity is not reduced, the test substance is found to have no such binding inhibitory activity.

In the present screening method, in addition to IRES and rpS5 (including the rpS5 in the form of serving as a portion of 40S ribosomal subunit, or a 80S ribosome fraction) other elements for detecting inhibitory effect against binding between IRES and rpS5 may be employed. Examples of such elements include a cytoplasm extract prepared from human cells or animal cells, such as HeLa cells; rabbit reticulocyte lysate which is used for in vitro translation reaction; a buffer; a variety of detection reagents; and diluents.

The milieu in which the present screening method is performed may be selected in accordance with the mode of the binding reaction between IRES and rpS5, and thus is not subject to any particular limitations. Specific examples include a well plate such as a 96-well plate, and a nitrocellulose membrane.

The present invention also provides a kit for performing the above-described present screening method; i.e., a kit for screening for an anti-HCV substance comprising, as components thereof, a component for providing IRES and a component for providing rpS5 (hereinafter the kit may be referred to as the present screening kit).

The present screening kit comprises, as essential components, a component for providing IRES (this component may be IRES *per se,* or may be a vector harboring a gene for providing IRES, a transformant obtained through transformation by use of the vector, etc.), and a component for providing rpS5 (this component may be not only rpS5 *per* *se* or an rpS5-containing material including the rpS5 in the form of serving as a portion of 40S ribosomal subunit, a 80S ribosome fraction, or a cytoplasm extract prepared from human cells or animal cells, such as HeLa cells, but also an amplification primer for amplifying a gene coding for rpS5, a vector harboring a gene coding for rpS5, a transformant obtained through transformation by use of the vector, an artificial oligopeptide prepared through chemical synthesis on the basis of the amino acid sequence of rpS5, etc.), and, as optional components, any of the following: a component for detecting inhibitory effect against binding between IRES and rpS5, such as rabbit reticulocyte lysate, which is used for in vitro translation reaction; a buffer; any of a variety of detection reagents; and diluents. The kit may also include means for enabling carrying out of the present screening method; e.g., a well plate such as a 96-well plate, and a nitrocellulose membrane. Preferably, these optional components are selected in accordance with the method for detecting inhibition against binding of IRES to rpS5 by a test substance (a specific embodiment will described below).

When the present screening method is performed by use of the present screening kit, effective screening can be performed for a substance exhibiting anti-HCV activity.

### Examples

The present invention will next be described by way of examples.

### [Test Example] Investigation of HCV-binding protein

### Preparation of plasmids

Plasmid pUC5END-nc (full-length cDNA of HCV 5'NCR being provided on the downstream region of T7 promoter) disclosed in Fukushi *et al*., J. Virol., 71, 1662-1666 (1997) was employed in the Test Example. cDNA of human rpS5 was prepared through selection of a fragment from a HeLa cDNA library and PCR amplification of the fragment. HS51 (5'-GGATCCGATGACGATGACAAAATGACCGAGTGGGAGAC-3': SEQ ID NO: 2) and HS52 (5'-GAAGCTTTCAGCGGTTGGACTTGGCCAC-3': SEQ ID NO: 3) were employed as PCR primers, and the resultant amplification product was treated with *Bam*HI and *Hind*III and inserted into a pQE 30 vector (product of Qiagen), to thereby obtain a plasmid pQE-RS5. In a manner similar to that for preparing pQE-RS5, pQE-RS9 (containing cDNA of human rpS9) was prepared from a PCR amplification product obtained by use of PCR primers HS91 (5'-GGATCCGATGACGATGACAAACCAGTGGCCCGGAGCTGGGTT-3': SEQ ID NO: 4) and HS92 (5'-GAAGCTTTTAATCCTCCTCCTCGTCGTC-3': SEQ ID NO: 5).

### In vitro RNA transcription reaction and UV crosslinking

[³²P]UTP-labeled probe of highly specific activity and corresponding to the 1st to 347th nucleotide of HCV 5'NCR was RNA-transcribed by use of an Riboprobe T7 System (product of Promega). The protein-RNA binding after performance of UV crosslinking was analyzed through the aforementioned method (the method disclosed in Fukushi *et al*., J. Virol., 71, 1662-1666 (1997)).

### Purification and characterization of p25

A cytoplasmic S10 extract was prepared from suspension-cultured HeLa S3 cells. The level of purification of p25 protein (HCV-binding protein, see Japanese Patent Application Laid-Open (*kokai*) No. 10-204100) was monitored on the basis of UV crosslinking of chromatographic protein fractions with a [³²P]UTP-labeled HCV RNA probe. Specifically, a HeLa S10 extract containing protein in an amount of approximately 650 mg was fractionated through gel filtration by use of Sephacryl S-300 (product of Amersham Pharmacia Biotech) which had been equilibrated in advance by use of buffer A (10 mM HEPES-KOH, pH 7.5, 1.5 mM MgCl₂, 1 mM dithiothreitol, and 5% glycerol) containing 120 mM KCl. Binding activity of p25 to an HCV 5'NCR RNA probe was confirmed near the void fraction. The results indicate that the p25 which is bound to an HCV IRES emerges as a portion of a macromolecule complex rather than a single cytomolecule.

The above void fraction was diluted with buffer A, and the diluted product was treated with a heparin-sepharose column [HiTrap heparin column (product of Amersham Pharmacia Biotech)] which had been equilibrated in advance by use of buffer A. Elution was carried out stepwise by use of buffer A of different KCl concentrations; specifically, 0, 150, 300, and 500 mM, thereby yielding fractions. Fractions exhibiting binding activity of p25 with respect to the HCV 5'NCR RNA probe were obtained through elution by use of buffers A of KCl concentrations of 300 mM and 500 mM. The resultant 500 mM KCl fraction was diluted 5-fold with buffer A, and the diluted fraction was subjected to cation-exchange chromatography by use of a column [Mono S HR 5/5 column (product of Amersham Pharmacia Biotech)] which had been equilibrated in advance by use of buffer A. Elution was carried out by use of buffer A while the KCl-concentration was gradually modified from 100 to 500 mM. Fractions exhibiting remarkable binding activity with respect to HCV 5'NCR RNA were obtained through elution by use of buffer A having a KCl concentration falling within a range of 250 to 340 mM (Figs. 2A and 2B, with silver staining images being provided in Fig. 2B) and stored. From the fractions, a first purified protein in an amount of approximately 38 mg was obtained.

### Analysis of amino acid sequence

The thus-purified protein was isolated by use of 14% gel SDS-PAGE. After electrical transfer to poly vinylidene difluoride membrane, the separated proteins were visualized through silver staining (Ponceau S for staining). Through visualization, two bands were identified near 25 kDa, and the peptide corresponding to p25 was observed just between two bands (Fig. 2C: lane 4). Of lanes other than lane 4, lane 1 shows an electrophoresis pattern of HeLa S10 crude extract; lane 2 shows that of the void fraction of gel filtration; and lane 3 shows that of the 500 mM KCl fraction obtained by use of heparin affinity column. Gel portions containing the two different proteins were cut out and treated with achromobactor proteinase I (Lys-C), and the amino acid sequence of the fragment of each of the cut portions was determined. Among ten amino acids contained in a slowly mobilized band, nine of them coincided with 155th to 163rd amino acids of human rpS9 (a), while ten amino acids contained in a rapidly mobilized band coincided with 23rd to 32nd amino acids of human rpS5 (b).

### Antibodies

Rabbit antisera for rpS5, rpS9, and rpS26, among rat 40S ribosomal proteins, were prepared through purification on the basis of immunoadsorption. Immunospecificity of these antisera has already been known [Heise *et al*., Acta Biol. Med. Ger., 37, 1353-1362 (1978)]. In addition, a method for preparing a polyclonal antibody for PTB (polypyrimidine tract-binding protein) has already been known [Lutsch *et. al*., Biomed. Biochim. Acta, 42, 705-723 (1983)].

### Immunoblotting and immunoprecipitation

JM 109 cells were transformed by use of pQERS5 and pQERS9, respectively, and recombinant rpS5 and rpS9 were isolated in accordance with the manufacturer's manual of Ni-NTA agarose chromatograph (product of Qiagen). The recombinant ribosomal proteins were separated through gradient SDS-PAGE by use of 5-20% gel, and the separated proteins were employed as standard proteins for Western blotting.

Immunoprecipitation was performed by use of an antiserum for PTB and antibodies for rat ribosomal proteins [anti-rpS5 antibody, anti-rpS9 antibody, and anti-rpS26, which specifically recognize recombinant human rpS5, rpS9, and rpS26, respectively]. Fig. 3A shows the specificity of the above anti-rpS5 antibody and anti-rpS9 antibody obtained on the basis of Western blotting by use of recombinant human rpS5 and rpS9. In Fig. 3A, gel lane 1 represented by CBB shows a CBB-stained image of recombinant human rpS5 which has undergone electrophoresis, and gel lane 2 shows a CBB-stained image of recombinant human rpS9 which has undergone electrophoresis. Lanes 1 and 2 which are labeled anti-S5 in Fig. 3A show the results of immunoblotting, by use of the above anti-rpS5 antibody, of electrophoresed recombinant human rpS5 and rpS9, respectively. The results indicate that the aforementioned anti-rpS5 antibody has specificity to recombinant human rpS5. Lanes 1 and 2 which are labeled anti-S9 in Fig. 3A show the results of immunoblotting, by use of the above anti-rpS9 antibody, of electrophoresed recombinant human rpS5 and rpS9, respectively. The results indicate that the aforementioned anti-rpS9 antibody has specificity to recombinant human rpS9.

Immunoprecipitation was performed under stringent conditions in order to prevent non-specific precipitation that would otherwise result from protein agglutination during incubation with an antibody. Specifically, the extract of HeLa S10 which had been crosslinked to [³²P]UTP-labeled HCV IRES RNA was denatured by heat in a crosslinking buffer containing 0.5% SDS, and the denatured product was centrifuged. The resultant supernatant was diluted 5-fold by use of a sample buffer (25 mM Tris-HCl, pH 7.4, 150 mM NaCl, 0.5% Nonidet P-40, and 1 mM dithiothreitol). Subsequently, the diluted product was pre-incubated for 60 minutes with protein G-Sepharose which had not been bound to an antibody, and the resultant sample was centrifuged briefly. The resultant supernatant was incubated with protein G-Sepharose which had been bound to the antibody in advance. The resultant precipitates were washed six times with a sample buffer, and analyzed through SDS-PAGE and autoradiography.

As a result, crosslinked p25 (Fig. 3B: lane 1) was found to have immunoprecipitated by the anti-rpS5 antibody (Fig. 3B: lane 3) and have not immunoprecipitated by the anti-rpS9 antibody (Fig. 3B: lane 4).

The anti-PTB antibody―control antibody―induced precipitation of a single protein of 58 kDa, corresponding to the molecular weight of PTB [Fig. 3B: lane 2]. The anti-rpS26 antibody induced no precipitation [Fig. 3B: lane 5]. These results indicate that no non-specific precipitation occurs in the above test systems.

As described hereinabove, p25 protein crosslinked to an HCV IRES can be identified to be rpS5.

The affinity of rpS5 to an HCV IRES is related with efficiency of initiation of translation of HCV RNA, indicating that this protein plays an important role in HCV translation. Furthermore, interaction between rpS5 and an IRES has specificity in HCV. Thus, an important role of rpS5 in HCV translation is attributable to promotion of binding between the 40S subunit and an IRES. This mechanism differs from recycling of 40S subunit during RNA translation occurring in typical cells.

### [Working Examples]

From the findings obtained in the above-described Test Examples, the present inventors have attained the present screening method and the kit used for screening. Specific embodiments of the screening method and the kit will next be described as working examples.

### (1) IRES and rpS5

### IRES

### (i) Full-length IRES

When RNA containing full-length HCV5'NCR is used as the IRES, there is employed a synthesized RNA having full-length HCV5'NCR and prepared through in vitro transcription reaction by use of cDNA as a template. The cDNA is prepared by use of the above-described plasmid pUC5END-nc having a T7 promotor, which has full-length HCV5'NCR cDNA on a downstream side of the promotor.

If necessary, the IRES can be labeled. The IRES may be labeled with ³²P among the above radioisotopes by use of a conventional method [for example, Sambrook *et al*., Molecular Cloning: A Laboratory Manual (2nd Edition), Cold Spring Harbor Laboratory Press (1989)].

A radioisotope substance other than ³²P; e.g., ³³P, ³⁵S, ¹²⁵I, or ¹³¹I mentioned above, may be used for labeling the IRES through a conventional method.

When a full-length IRES is employed as a component of the present screening kit, the plasmid pUCSEND-nc is used. Optionally, a T7 RNA synthase and a buffer for synthesis (400 mM Tris-HCl (pH 8.0) containing 80 mM magnesium chloride, 20 mM spermidine, and 50 mM dithiothreitol) may also be employed as components thereof.

### (ii) Synthesized IRES

In the present example, RNA which has been chemically synthesized may be employed as the synthesized IRES. The RNA is a potion of stem-loop region II within HCV5'NCR. The RNA has bases corresponding to the 47th to 67th nucleotide sequence in the sequence represented by SEQ ID NO: 1.

If necessary, the IRES can be labeled. The IRES may be labeled with ³²P by use of a conventional method (kination reaction) [for example, Sambrook *et al*., Molecular Cloning: A Laboratory Manual (2nd Edition), Cold Spring Harbor Laboratory Press (1989)].

When a synthesized IRES is employed as a component of the present screening kit, a solution of the synthesized IRES or a freeze-dried product thereof may be used. Optionally, a T4-polynucleotide kinase for labeling and a reaction buffer (500 mM Tris-HCl (pH 8.0) containing 100 mM magnesium chloride and 50 mM dithiothreitol) may also be employed as components thereof.

### rpS5

The rpS5 employed is any of the following (i) to (v). (i) a cytoplasm extract (the above-described HeLaS3 extract); (ii) 80S ribosome obtained through the above-described routine method in which the cytoplasm extract is subjected to ultra centrifugation; (iii) a 40S ribosomal subunit obtained through a process in which the 80S ribosome is subjected to treatment with KCl and treatment with puromycin, and then to purification by use of sucrose density-gradient centrifugation or a similar means; (iv) rpS5 isolated and purified by use of a customary method, such as ion exchange column chromatography, from the 40S ribosomal subunit; and (v) recombinant rpS5 isolated from a recombinant JM109 cell which is a transformant obtained by use of pQERS5, as described above.

When rpS5 is employed as a component of the present screening kit, modes of the rpS5 include: a dispersion of any of the above-described rpS5 of (i) to (v) in phosphate bufferred saline; a freeze-dried product thereof; and the pQERS5 described in (v) (as described above, recombinant rpS5 can be isolated after JM109 cells or similar cells are subjected to transformation).

### (2) Detection method

### Illustrative embodiment in which the UV cross linking method is employed

1) The IRES is (i) a labeled full-length IRES (alternatively, a synthesized IRES may be employed).
2) The rpS5 is (i) a cytoplasm extract, (ii) 80S ribosome, or (iii) a 40S ribosomal subunit (alternatively, rpS5 of (iv) or (v) may be employed).
3) A binding buffer (720 mM potassium chloride, 12.5 mM magnesium chloride, 38% glycerol, 7.5 mM adenosine triphosphate, and 10 mM guanosine triphosphate) is employed.
4) A sample dilution buffer (10 mM HEPES (pH 7.5), 1.5 mM magnesium acetate, and 1 mM dithiothreitol) is employed.
5) A non-specific RNA competitive reagent (containing poly (I) (2 mg/ml) and poly (I) (C) (2 mg/ml)) is employed.
6) For UV irradiation, a Terasaki plate is employed.
7) An RNA decomposition enzyme (10 mg/ml) is employed.

In the binding buffer which has been diluted 10-fold, the following substances are mixed: the labeled IRES, the rpS5 which has been diluted with or dissolved in the sample dilution buffer, the non-specific RNA competitive reagent, and a test substance. The mixture is incubated at 30°C for 10 minutes. Subsequently, the incubated mixture is transferred to the Terasaki plate for UV-irradiation, and the plate is irradiated with UV light on ice for 15 minutes. Then, the RNA decomposition enzyme is added thereto, followed by incubation at 37°C for 15 minutes. The resultant mixture is electrophoresed on an SDS polyacrylamide gel. After completion of electrophoresis, the gel is dried with a gel drier, and the dried gel is subjected to autoradiography or similar analysis. Anti-HCV activity of the test substance is determined on the basis of whether or not a signal is detected at the position corresponding to the molecular weight of rpS5.

If a signal is detected at the position corresponding to the molecular weight of rpS5, the test substance is considered to have no effect of inhibiting binding of rpS5 to IRES. In contrast, if a signal is not detected at the position corresponding to the molecular weight of rpS5, the test substance is considered to have an effect of inhibiting binding of rpS5 to IRES.

Binding of rpS5 to IRES is indispensable for initiation of translation of HCV gene. Therefore, if a test substance has an effect of inhibiting binding of rpS5 to IRES, the test substance is capable of preventing multiplication of HCV in a host by means of inhibiting translation of the virus gene. Thus, it is possible to detect whether or not a test substance has anti-HCV activity based on inhibition of HCV multiplication, so test substances can be successfully screened for a substance exhibiting anti-HCV activity.

A kit for the above-described embodiment of the present screening method includes the following two essential components: 1) a component for providing IRES, and 2) a component for providing rpS5. In addition, the kit may optionally include 3) a binding buffer, 4) a sample dilution buffer, 5) a non-specific RNA competitive reagent, 6) a Terasaki plate for UV-irradiation, and/or 7) RNase, as optional components for the UV cross linking method.

### Illustrative embodiment in which a gel shift assay is employed

1) The IRES is (ii) a synthesized IRES that has been labeled (alternatively, a full-length IRES may be employed).
2) The rpS5 is (iv) purified rpS5 (alternatively, recombinant rpS5 as described in (v) or rpS5 as described in any of the above (i) to (iii) may be employed).
3) A binding buffer (720 mM potassium chloride, 12.5 mM magnesium chloride, 38% glycerol, 7.5 mM adenosine triphosphate, and 10 mM guanosine triphosphate) is employed.
4) A sample dilution buffer (10 mM HEPES (pH 7.5), 1.5 mM magnesium acetate, and 1 mM dithiothreitol) is employed.
5) A specific RNA competitive reagent (containing poly (I) (2 mg/ml) and poly (I) (C) (2 mg/ml), tRNA (15 mg/ml), and 18S ribosomal RNA (15 mg/ml)) is employed.

In the binding buffer which has undergone 10-fold dilution, the following substances are mixed: the labeled IRES, the rpS5 which has been diluted with or dissolved in the sample dilution buffer, the specific RNA competitive reagent, and a test substance. The mixture is incubated at 30°C for 10 minutes. Subsequently, the incubated mixture is electrophoresed for separation on an polyacrylamide gel under conditions of non-denaturation. After completion of electrophoresis, the gel is dried with a gel drier, and the dried gel is subjected to autoradiography or similar analysis. Difference in electrophoresis migration distance between the incubated mixture and RNA which is not bound to a protein is investigated.

Any difference in electrophoresis migration distance between the incubated mixture and RNA which is not bound to a protein indicates that a complex of rpS5 and IRES has been formed, and therefore, the test substance is considered to have no effect of inhibiting binding of rpS5 to IRES. On the other hand, if no difference in electrophoresis migration distance is observed, a complex of rpS5 and IRES has not been formed, and therefore, the test substance is considered to have an effect of inhibiting binding of rpS5 to IRES.

Binding of rpS5 to IRES is indispensable for initiation of translation of HCV gene. Therefore, if a test substance has an effect of inhibiting binding of rpS5 to IRES, the test substance is capable of preventing multiplication of HCV in a host by means of inhibiting translation of the virus gene. Thus, it is possible to detect whether or not a test substance has anti-HCV activity based on inhibition of HCV multiplication, so test substances can be successfully screened for a substance having anti-HCV activity.

A kit for the above-described embodiment of the present screening method includes the following two essential components: 1) a component for providing IRES, and 2) a component for providing rpS5. Moreover, the kit may include 3) a binding buffer, 4) a sample dilution buffer, and/or 5) a specific RNA competitive reagent, as optional components for the gel shift assay.

### Illustrative embodiment in which the surface plasmon resonance method is employed

1) The IRES is (i) a labeled full-length IRES (alternatively, a synthesized IRES may be employed).
2) The rpS5 is (v) recombinant rpS5 (purified rpS5 of (iv) or rpS5 as described in any of the above (i) to (iii) may be employed).
3) A coupling buffer (10 mM HEPES (pH 7.5), 150 mM KCl, and 0.05% Surfactant P20) is employed.
4) An amine coupling kit is employed.
5) A sensor chip is employed.

IRES is immobilized onto the sensor chip by use of the amine coupling kit, and the chip is set on a flow cell of a surface plasmon resonance assaying apparatus. rpS5 dissolved in the coupling buffer is added to the flow cell through a sample port of the apparatus. Subsequently, a test substance dissolved in the coupling buffer is also added to the flow cell through the sample port. Binding of rpS5 to IRES causes change in mass of the substances present on the sensor chip, and the change is detected as an SPR signal, which is employed to monitor the formation of a complex of rpS5 and IRES.

When a test substance is added, if no SPR signal change is detected, the test substance is considered to exhibit no effect of inhibiting binding of rpS5 to IRES. In contrast, if any SPR signal change by addition of a test substance is detected, the test substance is considered to have an effect of inhibit binding of rpS5 to IRES.

Binding of rpS5 to IRES is indispensable for initiation of translation of HCV gene. Therefore, if a test substance exhibits an effect of inhibiting binding of rpS5 to IRES, the test substance is capable of preventing multiplication of HCV in a host by means of inhibiting translation of the virus gene. Thus, it is possible to detect whether or not a test substance has anti-HCV activity based on inhibition of HCV multiplication, so test substances can be successfully screened for a substance having anti-HCV activity.

A kit for the above-described embodiment of the present screening method includes the following two essential components: 1) a component for providing IRES, and 2) a component for providing rpS5. In addition, the kit may further include, as optional components, 3) a coupling buffer, 4) an amine coupling kit, and/or 5) a sensor chip, in order to carry out the surface plasmon resonance method.

## Claims

1. A method for screening test substances for a substance exhibiting anti-hepatitis-C-virus activity, through detection of inhibitory activity of the substance against binding between an IRES of a hepatitis C virus gene and a 40S ribosomal protein S5.

2. The method according to claim 1, wherein the 40S ribosomal protein S5 is in a state in which it composes a portion of a 40S ribosomal subunit.

3. The method according to claim 1, wherein the 40S ribosomal protein S5 is in a state in which it composes a portion of a 80S ribosome.

4. A kit for screening for an anti-hepatitis-C-virus substance comprising, as components thereof, a component for providing IRES of a hepatitis C virus gene and a component for providing a 40S ribosomal protein S5.

5. The kit according to claim 4, wherein the component for providing a 40S ribosomal protein S5 is a component for providing a 40S ribosomal protein S5 which is in a state in which it composes a portion of a 40S ribosomal subunit.

6. The kit according to claim 4, wherein the component for providing a 40S ribosomal protein S5 is a component for providing a 40S ribosomal protein S5 which is in a state in which it composes a portion of a 80S ribosome.


SEQUENCE LISTING

<110> BML, INC.
      Max-Delbrück-Centrum für Molekulare Medizin

<120> Method of Screening Drug for Hepatitic C

<130> PBM65/EP

<140>
<141>

<150> JP P2001-329728
<151> 2001-10-26

<160> 5

<170> PatentIn Ver. 2.1

<210> 1
<211> 347
<212> DNA
<213> Hepatitis C virus

<400> 1
gccagccccc tgatgggggc gacactccac catgaatcac tcccctgtga ggaactactg 60
tcttcacgca gaaagcgtct agccatggcg ttagtatgag tgtcgtgcag cctccaggac 120
cccccctccc gggagagcca tagtggtctg cggaaccggt gagtacaccg gaattgccag 180
gacgaccggg tcctttcttg gataaacccg ctcaatgcct ggagatttgg gcgtgccccc 240
gcaagactgc tagccgagta gtgttgggtc gcgaaaggcc ttgtggtact gcctgatagg 300
gtgcttgcga gtgccccggg aggtctcgta gaccgtgcac catgagc 347

<210> 2

<211> 38
<212> DNA
<213> Homo sapiens

<400> 2
ggatccgatg acgatgacaa aatgaccgag tgggagac 38

<210> 3
<211> 28
<212> DNA
<213> Homo sapiens

<400> 3
gaagctttca gcggttggac ttggccac 28

<210> 4
<211> 42
<212> DNA
<213> Homo sapiens

<400> 4
ggatccgatg acgatgacaa accagtggcc cggagctggg tt 42

<210> 5
<211> 28
<212> DNA
<213> Homo sapiens

<400> 5
gaagctttta atcctcctcc tcgtcgtc 28